# EUROPEAN PATENT SPECIFICATION

(11) **EP 3 419 590 B1**
(45) Date of publication and mention of the grant of the patent: **27.01.2021**
(21) Application number: 17716603.0
(22) Date of filing: 20.02.2017
(51) Int. Cl.: A61K 8/73, A61Q 19/00

(54) **HYALURONIC ACID CONJUGATE**
HYALURONSÄUREKONJUGAT
CONJUGUÉ D'ACIDE HYALURONIQUE

(30) Priority: 26.02.2016 IT UB20161125
(43) Date of publication of application: 02.01.2019
(73) Proprietor: Ribohyal S.r.l., 00144 Rome (IT)
(72) Inventor: BARICORDI, Nikla, 45030 Occhiobello (RO) (IT); CARUSO, Ciro, 80128 Napoli (IT)
(74) Representative: Di Giovine, Paolo
(86) International application number: PCT/IB2017/050943
(87) International publication number: WO 2017/145035

(56) References cited:
- WO-A1-2015/071873
- WO-A1-2016/153893
- WO-A1-2016/153910
- WO-A2-2016/160347
- RU-C2- 2 386 640
- DATABASE WPI Week 201034 Thomson Scientific, London, GB; AN 2010-F35170 XP002764302, & RU 2 386 640 C2 (AS USSR SYNTHETIC POLYMER MATERIALS INST) 20 April 2010 (2010-04-20)

## Description

### Technical field of the invention

The present invention relates to compositions comprising hyaluronic acid conjugated to riboflavin, for use according to the claims, in cosmetic and therapeutic field.

The object of the present invention is to provide new hyaluronic acid-based compositions solving the disadvantages linked to the use of the current hyaluronic acid-based compositions.

### State of art

The treatments linked to the use of *fillers* to correct face imperfections have become more and more frequent during the last years, by finding specific indications in different situations and by representing a valid alternative to not always accepted or possible surgical operations, such as front lifting, malar plastics (zygoma surgical augmentation), chin plastics and cheiloplasty (lip augmentation).

The first used fillers, apart from liquid silicone, illegal by now for many years, were bovine collagen-based fillers and they were introduced in the clinical practice in the 80s; they allowed good results but they needed preliminary allergy tests. Subsequently other substances were studied with the purpose to avoid the preliminary tests and to increase product residence and effectiveness. Depending upon time duration, schematically one can speak about re-absorbable fillers, semi-permanent fillers and permanent fillers. The permanent fillers and even the semi-permanent ones may create, over time, even serious inflammatory reactions, they can move and reach the surface by causing problems and deformities which not always can be controlled which limit hugely the use thereof for aesthetical reasons.

For these reasons, by now for several years the more frequently used fillers have been hyaluronic acid-based fillers, wholly biocompatible and without the risk of allergies, re-absorbable, which do not involve inflammatory reactions after long time.

The fillers can be used with different purposes in order to obtain a filling effect that is to augment volumes. In the first application, they reduce or eliminate both the surface and deep wrinkles apart from the depressions of the skin thickness of different origin: the injection below the defect relaxes it and levels it in much natural way. On the contrary, when they are used to augment volumes they are applied in different seats. The lips can be improved by highlighting the contour and the design thereof, by making them more harmonious as a whole, without modifying the volumes thereof, that is they can be augmented when they are too thin. The mouth angles can be filled-in and raised by giving a more relaxed and calm appearance to the face and by eliminating the sad appearance. Zygoma can be augmented by giving more character to the face by providing to it a younger appearance. The chin can be modified both in shape (for example by rounding it if too pointed and viceversa) and in the projection (in case of a receding chin). The mandibular contour can be modified by harmonizing the profile above all in the front portion or by modifying slightly the oval shape by highlighting the region of the rear angle by making the face more "volitive". A greater re-integration of the volumes with more homogeneous distribution of the products extended even to the cheeks with a "rejuvenating effect and not surgical medical lifting" can be obtained by using greater quantities of product by injecting them with suitable microcannules. Products of different type and with different composition can be used according to the seat.

The hyaluronic acid with more than 10 millions of performed treatments currently is the most frequently used substance as "*filler*", that is "filling substance", under such term by indicating the injectable implants used to correct winkles, furrows, depressions and for augmenting the volume of lips, zygomatic regions, mandibular contour and of other face areas. The hyaluronic acid is a natural substance, usually included in our organism, as component of the extra-cellular matrix; it is important for forming the matrix of collagen and elastic fibres and for maintaining the skin hydration. In dermis, in fact, it plays the function of supporting scaffolding by linking to other substances and giving compactness to the skin and hydration since it has high capability to link water. The alterations of the hyaluronic acid contribute to the skin ageing. The action mechanism of the injected hyaluronic acid as *"filler"* is due to the viscoelastic properties of the product, to the capability of drawing and keeping water and to the ex novo synthesis of collagen thanks to the stimulation of fibroblasts. The hyaluronic acid-based *fillers* mainly are constituted by a crystalline gel of hyaluronic acid, synthetized in laboratory with different formulations even in relation to the seats wherein the product is injected and to the type of requested result. It is a sterile, transparent and viscoelastic gel. Once injected, the hyaluronic acid is gradually re-absorbed by the organism. As it decomposes, its place is occupied by water. When it is wholly absorbed, it transforms into water and carbon dioxide and it disappears from the body without leaving any trace. The hyaluronic acid is synthetized in laboratory and it is not of animal origin; for these reasons there are no risks of triggering allergic reactions in case one is sensible to common food such as beef, chicken and eggs or disease transmission.

In the last years even a hyaluronic acid-based product crosslinked by the crosslinking agent BDDE (US 8450475) has been developed. The use thereof is listed among the less invasive aesthetical techniques to reduce wrinkles. The technique with which this substance is inserted below the skin is the one of the fillers which can have good and long-lasting results in case the crosslinked hyaluronic acid is used. However, the use of this substance offers temporary results, therefore to prolong the effects it is necessary to be subjected periodically to this type of treatment.

The patent application RU2386640 describes a process for preparing a complex constituted by a crosslinked hyaluronic acid salt, modified with riboflavin, characterized in that the chemical interaction of the hyaluronic acid-riboflavin salt complex takes place by means of at least a crosslinking agent and it is performed, by exposing the initial reagents to a pressure in the range from 5 to 1000 MPa in a mechanical-chemical reactor at a temperature between 20 and 50°C.

The patent application WO2015/071873 describes a nanohydrogel constituted by riboflavin derivatized with an alkyl group suitable to be linked covalently to a polysaccharide.

Then, the problem of providing new *fillers,* not having the disadvantages of the *fillers* known in the state of art, is much felt.

### Summary of the invention

The present invention relates to a composition for use according to the claims, comprising a hyaluronic acid conjugated with riboflavin or derivatives thereof. The conjugate according to the present invention, differently from the other known products, will be subjected to crosslinking after having been injected in dermis and in natural way. The conjugate according to the present invention further has the following important properties:
1 it absorbs UVA and UVB by preventing from damaging the by now known insult thereof on the collagen fibres of dermis;
2 it produces a natural crosslinking both a) by forming a net of collagen fibres by reinforcing and strengthening the dermis and b) by reducing and neutralizing selectively the production of free radicals responsible for skin and cutaneous ageing and c) by stimulating the collagen neosynthesis with consequent longer duration.

Other advantages and features and use modes of the present invention will result evident from the following detailed description of some embodiments and from the experimental data reported in the section of examples, shown by way of example and not for limitative purposes.

### Detailed description of the invention

The present invention firstly relates to a composition for use according to the claims, comprising a hyaluronic acid conjugated at least with riboflavin or a derivative thereof, wherein the hyaluronic acid is conjugated to riboflavin by a covalent bond and wherein said covalent bond is an ester bond between the carboxylic group of hyaluronic acid and the C20 carbon of riboflavin according to the structure formula (I):

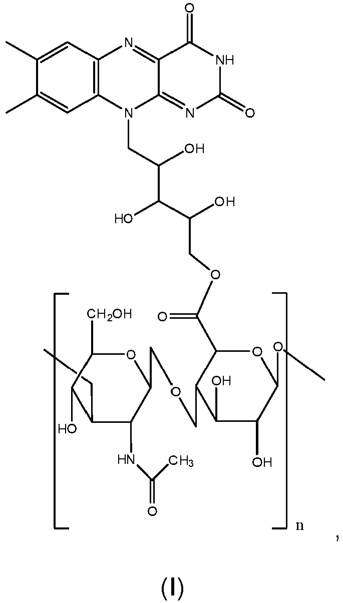

As used herein, the term hyaluronic acid (HA) can mean any hyaluronic acid or salts thereof, and it includes, but it is not limited to them, sodium hyaluronate (NaHA), potassium hyaluronate, magnesium hyaluronate, calcium hyaluronate, and combinations thereof.

In the present description under the term derivative of riboflavin (or vitamin B₂) any derivative of riboflavin is meant, for example flavin mononucleotide (FMN), flavin adenine dinucleotide (FAD). Other examples of derivatives of riboflavin are constituted according to the formula shown hereinafter: 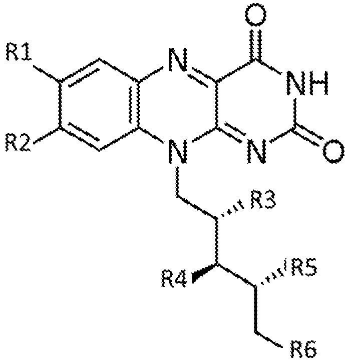 wherein R1, R2, R3, R4, R5 and R6 are independently selected among hydrogen, hydroxyl, methyl, optionally substituted C2-C6 alkynyl, optionally substituted aryl, optionally substituted C1-C6 alkyaryl, optionally substituted aryl C1-C6 alkyl, optionally substituted heteroaryl, optionally substituted C1-C6 alkylheteroaryl, optionally substituted heteroaryl C1-C6 alkyl, optionally substituted C2-C6 alkenylaryl, optionally substituted aryl C2-C6 alkenyl, optionally substituted C2-C6 alkenylheteroaryl, optionally substituted heteroaryl C2-C6 alkenyl, optionally substituted C3-C8-cycloalkyl, optionally substituted heterocycloalkyl, optionally substituted C1-C6 alkyl C3-C8-cycloalkyl, optionally substituted C3-C8-cycloalkyl C1-C6 alkyl, optionally substituted C1-C6 alkylheterocycloalkyl and optionally substituted heterocycloalkyl C1-C6 alkyl, alkyl, alkenyl, alkynyl, C3-C8-cycloalkyl, heterocycloalkyl, alkylaryl, alkylheteroaryl, alkylcycloalkyl, amino, aminosulphonyl, halogen, fluorine, chlorine, bromine and iodine or combinations thereof.

The hyaluronic acid is conjugated to riboflavin by a covalent bond. Said covalent bond is an ester bond between the carboxylic group of hyaluronic acid and the C20 carbon of riboflavin, said conjugation for example could be obtained by using carbodimide (EDC). The molecular formula of hyaluronic acid with riboflavin is (C31 H39 N5 O16)n and its IUPAC name is 5-(7,8-dimethyl-2,4-dioxo-3,4-dihydrobenzo[g]pteridin-10(2H)-il)-2,3,4-trihydroxypentyl 3-(((2R,3R,4R,5S,6R)-3-acetamido-4,5-diydroxy-6-(hydroxymethyl)tetrahydro-2H-piran-2-il)metoxy)-4,5,6-trihydroxytetrahydro-2H-piran-2-carboxylate. The structure formula representing the conjugate is the above mentioned structure formula (I), wherein the disaccharide unit is represented which repeats n times in the hyaluronic acid structure, for example wherein n is comprised between 10 and 1000000 times, or 100 and 10000 times.

According to an embodiment the degree of substitution of riboflavin in the esters is comprised between 2 and 7%, wherein the term "degree of substitution" designates the number of moles of riboflavin per mole of glycosaminoglycan.

According to an embodiment the esters obtained from conjugation of hyaluronic acid with riboflavin have a molecular weight comprised between 250.000 and 800.000 Dalton, under this interval by designating the average molecular weight of the glycosaminoglycan without considering the contribution of the weight of riboflavin.

The present invention relates both to conjugates wherein the hyaluronic acid is linked exclusively to riboflavin and conjugates wherein the hyaluronic acid or riboflavin is linked also to other molecules, for example to other vitamins. The expression hyaluronic acid conjugated at least with riboflavin in the present description then designates both a conjugate consisting of hyaluronic acid linked to a covalent bond to riboflavin and a conjugate comprising hyaluronic acid linked to riboflavin wherein the hyaluronic acid and/or riboflavin can be linked even to other molecules.

The present invention also relates to a method for the preparation of the hyaluronic acid conjugated with riboflavin comprising a step of activating the carboxylic function of hyaluronic acid with a carbodiimide (EDC). After activation, the hyaluronic acid will react with riboflavin by forming the corresponding ester.

According to an embodiment the preparation method will provide a step wherein the hyaluronic acid salt, in particular sodium hyaluronate, is dissolved in water and the gel is brought to a *pH* comprised between 3.5 and 4.5 with HCI preferably 0.1N; the carboxylic function of hyaluronic acid is activated by using a carbodiimide (EDC), then it is made to react with riboflavin at a temperature comprised between 8 and 12°C. Riboflavin is not used as such, but, in order to dissolve it in water, it is transformed into a Boron complex, soluble in aqueous environment. The mixture is left under stirring for 1-2 hours, then the product is precipitated by adding sodium chloride and ethanol.

For the preparation of hyaluronic acid-riboflavin conjugate as starting material a hyaluronic acid with high or low molecular weight could be used, preferably hyaluronic acid obtained by bacterial synthesis (biofermentation) will be used which provides a lower possibility of allergic reaction than that of animal origin. Several methods for obtaining hyaluronic acid from bacterial synthesis are known to the person skilled in the art, as described for example in the patent EP01 37786. Even hyaluronic acid of animal origin, of chemical synthesis, crosslinked hyaluronic acid or combinations thereof could be used. Examples of hyaluronic acid crosslinked by the crosslinking agent BDDE are described in US 8450475.

The term hyaluronic acid with high molecular weight in the present document describes a hyaluronic acid having a molecular weight comprised between 1.0 millions of Daltons and about 4.0 MDa. For example, the high molecular weight of hyaluronic acid could have a molecular weight of about 2.0 MDa. In another example, it can have a molecular weight of about 2.8 MDa.

The term hyaluronic acid with low molecular weight in the present document describes a hyaluronic acid having a molecular weight lower than about 1.0 MDa. Hyaluronic acid with low molecular weight can have a molecular weight comprised between about 200,000 Da (0.2 MDA) and less than about 1.0 MDa, for example, between about 300,000 Da (0.3 M Da) and about 750,000 Da (0.75 MDA).

The present invention also relates to compositions comprising the hyaluronic acid conjugate according to anyone of the herein described embodiments and/or one or more carriers and/or excipients and/or diluents and/or active ingredients.

Depending upon the use the composition could have different formulations, for example in form of gel, suspension, solution (in particular aqueous solution), capsule, tablet, pill, granulate, powder, eye drops, shampoo, lotion, *fillers* (compositions which are injected in dermis or in the subcutaneous tissue), spray.

In an embodiment the composition as described herein, in anyone of the above-illustrated embodiments, could be in form of pharmaceutical composition, that is including ingredients of pharmaceutical grade or it could be or could be inserted in a medical device.

The composition according to the present description could be implemented in form of pharmaceutical composition or medical device according to anyone of the classes described in the Directive 93/42/EEC about the medical devices (which comprises even substances and not only "devices" in the mechanical sense of the term), or in form of food supplement or in any form according to the provisions regulating the country wherein such composition will be produced.

According to a preferred embodiment the composition will include the hyaluronic acid conjugate in a concentration between 2% and 3% by weight of the composition. According to a preferred embodiment the composition is a gel with hyaluronic acid conjugate and crosslinked with a viscosity between 200,000 and 300,000 mPa-s.

According to an embodiment the composition will include the conjugate in a hyaluronic acid/riboflavin ratio equal to 24 mg/ml / 1 mg/ml.

The present invention also relates to a kit comprising a composition according to anyone of the herein described embodiments in form of gel, suspension and/or one or more syringes, already filled in, or not, with such compositions, and/or one or more needles suitable for administering such compositions, for example needles: 27 G ½ or 30 G ½ according to the viscosity of the gel.

Its own feature of hyaluronic acid is the capability of being inserted very well in the tissues wherein it is injected and of being filled-in in a very natural way. In its formulation in gel, this substance allows a better filling and a good penetration level. This feature makes it suitable for filling grooves and depressions (nasolabial furrows, labio-chin furrows, nasojugal furrow) and the lips requiring a volume augmentation or a slight modification in the design. The product duration varies according to density and the structure of the product from a minimum of 4-6 months for fluid products (with low molecular weight and/or with short chain and/or less viscous) and surface corrections to a maximum of 12 months for consistent products (with high molecular weight and/or with long chain and/or more viscous) and deep corrections.

The method thereby the crosslinked acid is used to level wrinkles and imperfections is that of the *filler.* This technique consists in using syringes wherein the hyaluronic acid, in particular under the form of gel, is inserted.

As aesthetical treatment it is performed in out-patients department and in one single session which lasts about half an hour. The technique consists in injecting the gel of crosslinked hyaluronic acid inside the skin by means of micro injections. The areas usually selected for this type of treatment against wrinkles are zygoma, eye contour, lips and cheeks. Whoever is very sensitive to pain could use an anaesthetic ointment shortly before the injections.

The results can be appreciated immediately at the end of the treatment.

If injected correctly in the "right" quantities and in the suitable seats the results are very natural and they are not artefact at all. Furthermore it is possible to divide the operation in several sessions, so as to search for the wished effect gradually and thus to improve the results thereof. The result strongly depends upon the starting situation, as in all surgery and aesthetical medicine treatments. Therefore it is very important that the surgeon and the patient discuss about the possibilities and the limits of the wished results.

In case of slightly marked nasolabial wrinkles, with simple depression at the sides of the nostrils, the results can be very satisfying and they can be obtained in one single session. On the contrary, in case of strongly marked nasolabial wrinkles not only several phials, but in case even several sessions, will be necessary to obtain the wished result. It must be well understood that the fillers are not an alternative to a real lifting, but they can contribute to improve the global appearance of the face.

As far as the lips are concerned, it is possible to highlight the contour thereof by making the vermillion more harmonious and beautiful, by enlarging slightly the volume in a very natural way with one or two phials of product in one single session. In case of very small and thin lips, if one wishes a more significant change, while maintaining the right equilibrium and a natural appearance, it is necessary to use at least two phials and often to repeat the session after 2-3 weeks to obtain the wished result. In this way it is possible to check gradually the augmentation by avoiding abrupt changes in the appearance and by reducing the risk of inflammatory oedema.

In the amorphous matrix of a connective tissue the hyaluronic acid conjugate according to the present invention is responsible for keeping the hydration, turgescence, plasticity and viscosity level, as it positions in the space in an aggregated shape thus by absorbing a considerable number of water molecules. It is also capable of acting as cement substance and as anti-shock molecule as well as an effective lubricating agent (ex. in the synovial liquid) by preventing the tissue cells from damaging by physical stresses.

The extreme length of the molecule together with its high hydration level allows several polymers of hyaluronic acid conjugate to organize and form a structure of reticular type which has two main functions: to create a molecular scaffolding to keep shape and tone of the tissue; to operate as filter against the free widespread of particular substances, bacteria, infecting agents in the tissue. Only the substances having enough low molecular weight to be able to pass through the "meshes" of this net could spread freely in the tissue; all substances with greater molecular weight as well as bacteria or viruses will remain trapped in the net.

According to an embodiment the injections of hyaluronic acid conjugate according to the present invention are used together with injections of collagen proteins in surgery and aesthetical dermatology to remove wrinkles and to prevent the skin from ageing. In ear surgery the hyaluronic acid conjugate is used to regenerate perforated tympanic membranes, in ophthalmic surgery to produce artificial tear drops, eye drops of syringes for interventions on the eye vitreous body, in surgery of cataract, glaucoma, ocular transplants and furthermore as colouring agent, in arthrology as antiphlogistic lubricating agent and agent for preserving the synovial liquid of joints. Recent studies show that it can link to CD44 receptors of the cells of hair follicles by favouring the division thereof and the production of hair. It is also used against inflammations and ulcerative lesions of mouth (aphthae, stomatites, etc.), especially those as a consequence of chemotherapy and radiotherapy, by reducing immediately pain and by promoting the healing thereof. According to an embodiment the product appears in the form of gel, spray or mouth wash. For example the gel or spray will be used directly on the ulcerative areas, in case of persistent pain it can be used even several times per day without any contraindication or side effect, apart from specific allergies. In domestic dermatology the hyaluronic acid conjugate is the main component of several topical commercial products in the category of the *fillers* and/or face creams, to be used during day or more often during night. The excipients of the several compositions are very similar therebetween, but they can be used in different percentages known to the person skilled in the art. An example is hydrating agents, long-chain carboxylic acids (omega-3), sodium or potassium benzoate, alcohols and many others.

The hyaluronic acid conjugate can be used: in the eye vitreous humour, in the synovial liquid, in the skin, in the cartilage, in tendons, in the umbilical cord, in the aorta walls. The hyaluronic acid conjugate preferably of biotechnological origin both with filler effect and with effect for cutaneous use in the form of creams and/or gel inserts among wrinkles and cutaneous folds. Once positioned it exploits the physiological cutaneous transpiration by keeping water and consequently by increasing the epidermal hydration, it further augments the volume thereof; the size augmentation involves a filling of the cutaneous furrows, consequently a smoothing action of the skin thanks to a phenomenon of the natural crosslinking and to the crosslinking action of the conjugate according to the present invention.

The conjugate according to the present invention gives the products wherein it is incorporated an important anti-wrinkle and hydrating action with immediate sensory perception. The molecule is furnished with several studies and tests performed on volunteers wherein it is deduced that apart from an immediate sensory perception of the action of the cream and/or gel and/or of the *filler* already after one hour after the application there is a decrease in the evidence of wrinkles by 35%.

According to an embodiment the product in the form of cream and/or gel is reconstituted by a hyaluronic acid conjugate with very low molecular weight (1000 - 3000 dalton) capable of penetrating the skin and to carry out from inside the above-described functions. This specific molecular weight allows an easy absorption by the epidermis, so as to equilibrate the hydration thereof and to improve the stability of the skin thereof. This improves the cohesion of the cells and the barrier effect of the skin.

Even a cosmetic or therapeutic method for the treatment of wrinkles and other skin ageing signs are herein described, comprising the step of administering, in particular by intradermic route, the hyaluronic acid conjugate according to the present invention.

The sofar described compositions could be used as solutions and/or ophthalmic eye drops for lubricating, trophic, cytoprotective, cytoreparing use, they could be further used for the treatment of corneal ectasia thereamong keratoconus, both in form of eye drops and/or ophthalmic solutions both in already filled-in syringes to be used in the treatment of the corneal crosslinking as photosensitizing agent as photoprotecting agent of the ophthalmic structures, from cornea to retina.

According to an embodiment the conjugate of the present invention will be used in an eye drop in association with drugs or natural preparations to be administered in the eye in the form of drops. For example in eye drops intended to treat ophthalmic pathologies (ex. glaucoma, uveitis, etc.) or intended to heal ocular disorders of not pathological nature (ex. red eyes, dry eyes etc.). Other examples are:
Antihistaminic eye drops - decongestive reinforcing action, designated for the treatment of seasonal allergies and the allergic reactions to drugs or other substances.

Eye drops against ocular dryness: these types of eye drops are enriched with moistening substances, necessary to guarantee the right humidity level to the eye.

Eye drops against red eyes: these formulations usually do not request prescriptions, as they are intended to heal a transitory disorder, linked for example to air insults or to penetration of a foreign body in the eye (ex. eyelash or contact lens). Eye drops enriched with steroid active principles: they are strong ocular anti-inflammatory drugs, suitable in case of serious phlogistic (inflammatory) phenomena, which cannot be healed by other drugs with more moderate action.

Eye drops for the treatment of glaucoma: they are pharmacological preparations aimed at reducing the intraocular pressure. We briefly remind that glaucoma is a disorder characterized by a pressure alteration inside the eye.

Eye drops with mydriatic effect: these types of eye drops are mainly used for diagnostic purposes, with the aim of expanding the pupil to ease the examination of the ocular structures thereof. Sometimes they are used even in the decongestive therapy in the conjunctival context.

Eye drops with NCE protective action with anti UVA (ultraviolet radiations of type A and of type B, blue light, light spectrum range from 200nm to 500nm) properties .

According to an embodiment the eye drop to be used in the treatment of irritation, redness, congestion and ocular pruritus triggered by allergic, chemical or physical (fumes, powders, intense lights, solar or snow reflection) causes will be a solution in flask comprising apart form the herein described conjugate Tetryzoline hydrochloride, for example in a concentration of 50 mg for 100 ml; excipients for example camomile distilled water - distilled water of lime - of sodium phosphate dodecahydrate - potassium of hydrogen phosphate - sodium chloride - of sodium edetate - polysorbate 80 - benzalkonium choloride - purified water.

According to an alternative embodiment it will be a solution in single-dose container comprising apart from the herein described conjugate Tetryzoline hydrochloride, for example in a concentration of 50 mg fpr 100 ml; water; excipients for example of sodium phosphate dodecahydrate - sodium phosphate monobasic - of sodium edetate - sodium chloride - purified water.

Valid eye drops, suitably manufactured to fight ocular dryness (better defined as xerosis) have to imitate the features of the lachrymal mucus, they have to be slightly alkaline, isotonic with the lachrymal film and naturally without preservatives. To this purpose, the single-dose small ampoules of eye drops (to be used once) containing mucopolysaccharides (ex. hyaluronic acid conjugate), synthesis polymers (ex. polyvinyl alcohol, povidone, carbopol, macrogol) and mucomimetic substances such as galattoxiglucan, appear to be particularly useful.

In order to prevent ocular dryness sometimes it is recommended to apply the so-called artificial tears, a type of eye drops formulated with viscous substances capable of replacing the lachrymal liquid.

According to an embodiment recommended in the treatment of the dry eye syndrome the composition is a solution in flask wherein there is 1 ml of eye drops, the solution includes: Active principle: Hyaluronic acid conjugate sodium salt (2mg), Excipients: Sodium chloride - Potassium chloride - Sodium phosphate monobasic monohydrate - Disodium phosphate dodecahydrate - Purified water. The solution in single-dose container: Active principle: Hyaluronic acid conjugate sodium salt mg 2; Sodium chloride - Potassium chloride - Sodium phosphate monobasic monohydrate - Disodium phosphate dodecahydrate - Purified water.

Red eyes constitute a quite common condition after prolonged expositions to light sources (ex. tanning lamps, xeno headlights, etc.), atmospherical agents (air, sun, atmospheric pollution) or after long hours of work or intense reading.

Red eyes, burning sensation, lacrimation and dryness typical of these circumstances tend to solve when the cause which has determined the disorder is removed. However, in presence of particularly sensitive eyes, some types of eye drops can speed up the recovery: the ocular preparations enriched with decongestive and emollient substances, such as *Euphrasia* and Camomile, are to be preferred.

According to an embodiment the composition is a solution in flask: 1 ml of eye drops, the solution includes: Active principle: Hyaluronic acid conjugate sodium salt mg 2 , Euphrasia and Camomile.; Excipients: Sodium chloride - Potassium chloride - Sodium phosphate monobasic monohydrate - Disodium phosphate dodecahydrate - Purified water. According to an alternative embodiment the composition is a solution in single-dose container: Active principle: Hyaluronic acid conjugate sodium salt mg 2 Euphrasia and Camomile.; Sodium chloride - Potassium chloride - Sodium phosphate monobasic monohydrate - Disodium phosphate dodecahydrate - Purified water.

For the treatment of all serious inflammatory phenomena of the eye, the most suitable eye drops are those formulated with corticosteroids, in case supported by a topic therapy with antibiotic eye drops.

The formulation of the steroid eye drops is quite complex due to the poor solubility of the active principles in water: in order to obviate this drawback the drugs which are poorly soluble in aqueous solvent are administered under the form of suspension (instable thermodynamically systems, which tend to sedimentation and aggregations, requesting to be stirred before use).

The active principles more commonly used to prepare this type of eye drops are: Cortisone, Fluorometholone, hydrocortisone and Desametasone.

According to an embodiment the steroid eye drops are a solution in 1-ml flask containing: Active principle: Hyaluronic acid conjugate sodium salt mg 2 and (Fluorometholone or hydrocortisone or Desametasone); Excipients: Sodium chloride - Potassium chloride - Sodium phosphate monobasic monohydrate - Disodium phosphate dodecahydrate - Purified water. The solution in single-dose container: Active principle: Hyaluronic acid conjugate sodium salt mg 2 and (Fluorometholone or Hydrocortisone or Desametasone); Sodium chloride - Potassium chloride - Sodium phosphate monobasic monohydrate - Disodium phosphate dodecahydrate - Purified water.

The eye drops enriched with antibiotic active principles are suitable for the treatment of all infective forms of the eye in general and of the conjunctiva in particular. To this purpose the ANTIBIOTIC and ANTIVIRAL EYE DROPS are used especially in the treatment of bacterial/viral conjunctivitis and infective uveitis.

These types of eye drops, requesting medical prescription, are to be selected based upon the pathogen responsible for the infection. For example, for the treatment of an ocular infection supported by Pseudomonas pyocyanea the most suitable eye drop is the one formulated with gentamicin; the fusidic acid, instead, is recommended for bacterial conjunctivitis due to Staphylococcus. According to an embodiment the solution in flask: 1 ml of eye drops, the solution includes: Active principle: Hyaluronic acid conjugate sodium salt mg 2 and (ANTIBIOTIC, put as many as possible of them); Excipients: Sodium chloride - Potassium chloride - Sodium phosphate monobasic monohydrate - Disodium phosphate dodecahydrate - Purified water. In the solution in single-dose container: Active principle: Hyaluronic acid conjugate sodium salt mg 2 and (ANTIBIOTIC, put as many as possible of them); Sodium chloride - Potassium chloride - Sodium phosphate monobasic monohydrate - Disodium phosphate dodecahydrate - Purified water.

On the contrary, the most used antiviral eye drops are prepared with Diclofenac or Ketorolac, active substances belonging to the class of NSAIFs (NonSteroidal Anti-Inflammatory Drugs) useful to mitigate the infection symptoms. When the ocular infection is accompanied by a marked inflammation, it is recommended to combine the antibiotic treatment with another type of corticosteroid-based eye drops. There are even some types of eye drops including a mixture of antibiotic + anti-inflammatory active principles. Solution in flask: 1 ml of eye drops, the solution includes: Active principle: Hyaluronic acid conjugate sodium salt mg 2 and Diclofenac or Ketorolac, Excipients: Sodium chloride - Potassium chloride - Sodium phosphate monobasic monohydrate - Disodium phosphate dodecahydrate - Purified water.

According to an embodiment a solution in single-dose container: Active principle: Hyaluronic acid conjugate sodium salt mg 2 and Diclofenac or Ketorolac, Sodium chloride - Potassium chloride - Sodium phosphate monobasic monohydrate - Disodium phosphate dodecahydrate - Purified water.

### Eye drops for glaucoma

Glaucoma, defined as the silent thief of sight, is an insidious ocular pathology, difficult to be detected, as it begins with no perceptible symptom.

Generally, the first symptoms of the disease appear when the sight is jeopardized by now. The use of specific eye drops for the treatment of glaucoma is necessary to stop the course of the disease, by minimizing the damages for the sight. The most suitable types of eye drops to fight glaucoma are:
Beta-blockers, necessary to reduce the altered pressure inside the eye. These medical eye drops - requesting the medical prescription - act actively in the aqueous humour, by reducing the production thereof. The active principles most used to this purpose: Metoprolol, Timolol and Betaxolol.

Analogous of PG (prostaglandins): after a specific treatment with this type of eye drops, the intraocular pressure reduces as the outflow of the aqueous humor. A typical side effect of these eye drops is the hyperpigmentation of the iris.

Sympathomimetic drugs (ex. Brimonidine, Pilocarpine): eye drops of second quality for the treatment of glaucoma. The typical side effects of these drugs (ex. abundant lacrimation, conjunctival hyperaemia) constitute a real obstacle to the therapy; then, these types of eye drops are not always suitable to treat the disease.

According to an embodiment the eye drops for treating the glaucoma will include the herein described conjugate and one of the active principles selected among Beta-blockers for example Metoprolol, Timolol and Betaxolol in suitable buffer for example phosphate buffer.

These pharmacological preparations - which can be found both in form of eye drops and as ointment or ophthalmic gel - are recommended for the treatment of eyes' infections and inflammations. The mydriatic eye drops generally are prepared with muscarinic antagonist drugs, such as Cyclopentolate, Homatropine, Phenylephrine and Atropine. By determining an evident dilatation of the pupil, this type of eye drops is suitable even to prevent the formation of rear synaechiae (pathological adherences which form in the inner structures of the eye) in the uveitis context.

According to an embodiment the mydriatic eye drops include the herein described conjugate and muscarinic antagonist drugs, such as Cyclopentolate, Homatropine, Phenylephrine and Atropine in suitable buffer for example phosphate buffer.

### EXAMPLES

### Example 1 Preparation of the complex of Boron of riboflavin (Riboflavin-B)

3.76g of Riboflavin are suspended in 40 ml of solution of NaOH 0.5M; 3.81g di sodium tetraborate are added to the suspension.

The mixture is brought to 40°C and left under stirring for 1 hour, during thereof all riboflavin dissolves.

The reaction mixture is poured into 200 ml of acetone, kept under stirring at room temperature: riboflavin-B precipitates, then it is filtered and vacuum-dried.

### Example 2 Preparation of an ester of hyaluronic acid with riboflavin

2.5 g of HA-Na are dissolved in 250 ml of distilled H2O.

The gel pH is brought to a value of 4 ± 0.5 by adding a solution of HCI 0.1N.

The gel is brought to a temperature of 10 ± 2°C by dipping into water and ice and it is kept under stirring; 0.2 eq of EDC and 1 eq of Riboflavin-B are added thereto in rapid sequence. The mixture is left cold under stirring for 1 hour and 30 minutes.

The reaction is interrupted by adding 25ml of saturated solution of sodium chloride. The product is precipitated by adding a volume of 96% ethanol; the supernatant is removed and the filtered residue is washed several times with ethanol in different concentrations, then it is vacuum-dried.

### Exemplifying formula

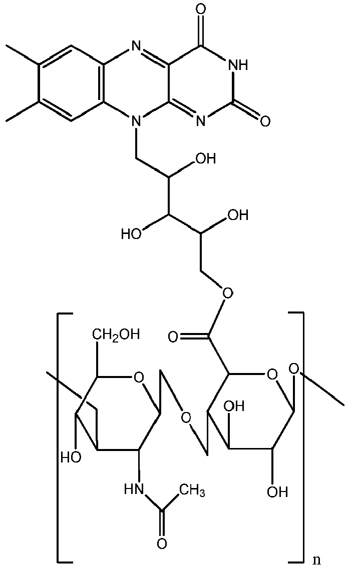

### Example 3 Preparation of an ester of hyaluronic acid with riboflavin

2.5 g of HA-Na are dissolved in 250 ml of distilled H2O.

The gel pH is brought to a value of 4±0,5 by adding a solution of HCI 0.1N. The gel is brought to a temperature of 10±2°C by dipping into water and ice and it is kept under stirring; 0.2 eq of EDC and 0.5 eq of Riboflavin-B are added thereto in rapid sequence. The mixture is left cold under stirring for 1 hour and 30 minutes. The reaction is interrupted by adding 25 ml of saturated solution of sodium chloride. The product is precipitated by adding a volume of 96% ethanol; the supernatant is removed and the filtered residue is washed several times with ethanol in different concentrations, then it is vacuum-dried.

### Example 4 Preparation of an ester of hyaluronic acid with riboflavin

2.5 g of HA-Na are dissolved in 250 ml of distilled H2O. The gel pH is brought to a value of 4±0.5 by adding a solution of HCI 0.1N. The gel is brought to a temperature of 10±2°C by dipping into water and ice and it is kept under stirring; 0.2 eq of EDC and 3 eq of Riboflavin-B are added thereto in rapid sequence. The mixture is left cold under stirring for 1 hour and 30 minutes. The reaction is interrupted by adding 25 ml of saturated solution of sodium chloride. The product is precipitated by adding a volume of 96% ethanol; the supernatant is removed and the filtered residue is washed several times with ethanol in different concentrations, then it is vacuum-dried.

The formation of crosslinks of HA with itself during the synthesis is possible. It is known that under the same reaction conditions in which the compound with riboflavin is produced, the formation of a cross-link of the hyaluronic acid with itself can take place. Even if this derivative is not quantified, it does not alter considerably the composition and the features of the end product.

### Example 5 evaluation of production of O-(singlet oxygen) by means of NMR

In order to evaluate the production of singlet oxygen by the herein described conjugate one has decided to insert the duly diluted substance into a cuvette which has been irradiated for preset periods of time with Xenon lamp XBO OSRAM 150 W OFR with polarized lens placed at 5 cm from the sample.

With the same dilutions a sample containing riboflavin only was then prepared. To evaluate the production of O-(singlet oxygen) as probe α-terpineol was selected which, by oxidizing, produces 8-hydroxycarvotanacetone. The compound was added to both solutions which have been mixed for 15 minutes before being irradiated.

As analytical technique one decided to use NMR, in order to perform a qualitative analysis of the oxidation: whereas in case of the herein described conjugate, the considerable decrease in the peaks of α-terpineol is observed, in case of riboflavin as such peaks of the not oxidised compound can be still observed.

### Example 6

Even if one does not want to link the present invention to any action mechanism, hereinafter a detailed description of the action mechanism of the combined action riboflavin + UVA rays in the herein described conjugate is shown.

The dermis has several layers of collagen fibres and the existence of so-called "cross-links" which increase the structural stiffness of the dermis itself.

The cross-linking (CXL) with riboflavin (vitamin B2), briefly designated with the word CXL, is a new technique for the treatment of tissues characterized by the presence of collagen fibres such as dermis and/or cornea and it consists in the combined administration of riboflavin and irradiation with ultra-violet rays (UVA) to strengthen the tissue itself subjected to such treatment.

The final goal of these preservative treatments is possibly to postpone and to stop the progressive depletion and degeneration of the collagen fibres. By using UVA light and with the protection of riboflavin new bonds between the adjacent collagen molecules are created so that the dermic tissue, full of collagen, reacquires stiffness.

The collagen "cross-linking" by means of Riboflavin and induced UV-A ray consists in the photo-polymerization of the fibrils of stromal collagen with the purpose of increasing the stiffness thereof and the resistance to the progressive tissue degeneration.

The collagen cross-linking aims at preventing and treating some physiopathological mechanisms, among the most important ones, causing ageing for having demonstrated:
- an effect of increasing resistance and biomechanical stability of the tissues full of collagen.
- an effect of increasing the fibrillar diameter of collagen
- a cell re-population starting from the deep layers (integer as to cells).

This re-polulating mechanism is the basis for the duration over time of the cross-linking phenomenon. In conclusion, the cross-linking can be a collagen reaction which determines an increase in the intra and interfibrillar connections with increase in stiffness and tissue resistance through the combination of a photosensible substance (riboflavin-vitamin B2) and the ultra-violet radiations of type A (UV-A). The Riboflavin solution has the function of absorbing the emitted UV-A light radiation and of exerting a physical function of protective barrier for the underneath structures. Such barrier effect is exerted above all in the front and intermediate layers of the dermic stroma. The combined application of Riboflavin (Vitamin B2)+UV-A (370nm, 3 mW/cm2) has the purpose of activating free radicals of oxygen (O2-) which induce an oxidative deamination of collagen and a consequent formation of new intrahelical and interfibrillar molecular bridges. Such effect can be quantified in an increase in the diameter in nm of collagen fibres on the average equal to 12% in the dermic stroma and in an increase in the tissue stiffness. The irradiated riboflavin, in the described method, has a double role:
- to allow the formation of a radical singlet oxygen (O2-);
- to absorb the emitted light radiation, in this way by allowing to concentrate the effect at a wished tissue depth and by protecting the deepest biological structures from irreversible damages.

Riboflavin, when it is hit by light, is subjected to a photolysis reaction which produces the detachment of a ribitol radical and the consequent loss of the vitamin action. The process of photopolymerization starts with an oxidative deamination and the formation of ribitol free radicals; they determine the formation of bonds between adjacent collagen filaments included inside the same fibril.

The present invention has been sofar described with reference to some preferred embodiments. It is to be meant that other embodiments may exist, all belonging to the same inventive core, as defined by the protection scope of the here below reported claims.

## Claims

1. A composition for use in a method of cosmetic surgery treatment, ophthalmic surgery treatment, or otologic surgery treatment or for use in a method of treatment of ulcerative lesions of the mouth comprising a hyaluronic acid conjugated at least with riboflavin or a derivative thereof, wherein the hyaluronic acid is conjugated to the riboflavin by a covalent bond and wherein said covalent bond is an ester bond between the carboxylic group of hyaluronic acid and the C20 carbon of riboflavin according to the structure formula (I):

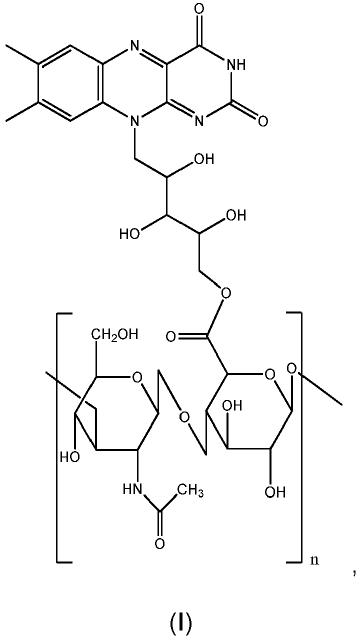

2. The composition for useaccording to claim 1 wherein the molecular weight of said hyaluronic acid conjugate is comprised between 200.000 and 5,000,000 of Dalton, in particular between 250.000 and 800.000 Dalton, wherein said molecular weight is the average molecular weight of hyaluronic acid without considering the contribution of the weight of riboflavin.

3. The composition for use according to claim 1 or 2 wherein in said hyaluronic acid conjugate the degree of substitution of riboflavin in the esters is between 2 and 7%.

4. The composition for use according to anyone of the preceding claims wherein said hyaluronic acid used for the conjugation with riboflavin is hyaluronic acid obtained by bacterial synthesis, hyaluronic acid of animal origin, crosslinked hyaluronic acid or combinations thereof.

5. The composition for use according to anyone of the preceding claims in form of gel, suspension, solution, capsule, tablet, pill, granulate, powder, eye drops, shampoo, lotion, *filler,* spray, mouthwash.

6. A method for the preparation of the hyaluronic acid conjugate according to anyone of claims 1 to 4 comprising a step of activating the carboxy groups of hyaluronic acid with a carbodiimide (EDC).

7. A kit for intra-dermal use comprising the composition according to claim 1 in form of gel, suspension or liquid and one or more syringes and/or one or more needles.

## Patentansprüche

1. Zusammensetzung zur Verwendung in einem Verfahren zur Behandlung in der Schönheits-, Augen- oder Ohrenchirurgie, oder zur Verwendung in einem Verfahren zur Behandlung von ulzerativen Läsionen des Mundes, die umfasst: eine Hyaluronsäure, die mindestens mit Riboflavin oder einem Derivat davon konjugiert ist, wobei die Hyaluronsäure mit dem Riboflavin durch eine kovalente Bindung konjugiert ist und wobei die kovalente Bindung eine Esterbindung zwischen der Carboxylgruppe der Hyaluronsäure und dem C20-Kohlenstoff von Riboflavin ist, gemäß der Strukturformel (I):

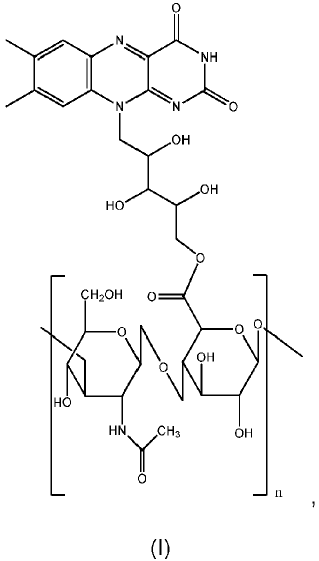

2. Zusammensetzung zur Verwendung gemäß Anspruch 1, wobei das Molekulargewicht des Hyaluronsäurekonjugats Werte zwischen 200.000 und 5.000.000 Dalton, im Besonderen zwischen 250.000 und 800.000 Dalton, umfasst, wobei das Molekulargewicht das durchschnittliche Molekulargewicht von Hyaluronsäure ohne Berücksichtigung des Beitrags des Gewichts von Riboflavin ist.

3. Zusammensetzung zur Verwendung gemäß Anspruch 1 oder 2, wobei der Substitutionsgrad von Riboflavin in den Estern in dem Hyaluronsäurekonjugat zwischen 2 und 7 % beträgt.

4. Zusammensetzung zur Verwendung gemäß einem der vorangehenden Ansprüche, wobei es sich bei der für die Konjugation mit Riboflavin verwendeten Hyaluronsäure um Hyaluronsäure handelt, die durch bakterielle Synthese erhalten wird, die tierischen Ursprungs ist, die vernetzt ist oder eine Kombination davon ist.

5. Zusammensetzung zur Verwendung gemäß einem der vorangehenden Ansprüche, die in den nachfolgenden Formen vorliegt: Gel, Suspension, Lösung, Kapsel, Tablette, Pille, Granulat, Pulver, Augentropfen, Shampoo, Lotion, Füllstoff, Spray, Mundwasser.

6. Verfahren zur Herstellung des Hyaluronsäurekonjugats gemäß einem der Ansprüche 1 bis 4, das umfasst: einen Schritt eines Aktivierens der Carboxylgruppen von Hyaluronsäure mit einem Carbodiimid (EDC).

7. Kit zur intradermalen Verwendung, das umfasst: die Zusammensetzung gemäß Anspruch 1 in Form eines Gels, einer Suspension oder einer Flüssigkeit und eine oder mehrere Spritzen und/oder eine oder mehrere Injektionsnadeln.

## Revendications

1. Composition pour une utilisation dans une méthode de traitement chirurgical cosmétique, de traitement chirurgical ophtalmique, ou de traitement chirurgical otologique, ou pour une utilisation dans une méthode de traitement de lésions ulcératives de la bouche, comprenant un acide hyaluronique conjugué au moins avec de la riboflavine ou un dérivé de celle-ci, dans laquelle l'acide hyaluronique est conjugué à la riboflavine par une liaison covalente et dans laquelle ladite liaison covalente est une liaison ester entre le groupe carboxylique de l'acide hyaluronique et le carbone C20 de la riboflavine conformément à la formule structurelle (I) :

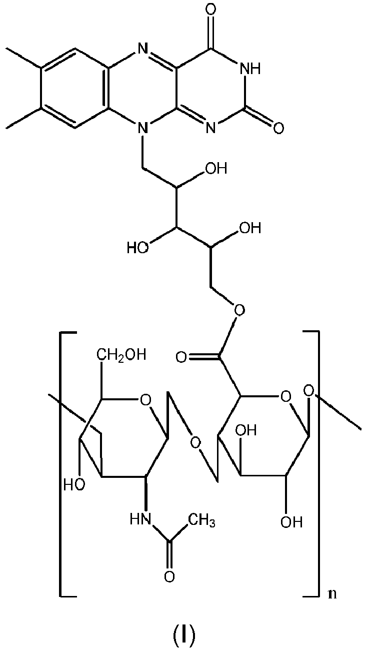

2. Composition pour une utilisation selon la revendication 1, dans laquelle la masse moléculaire dudit conjugué d'acide hyaluronique est comprise entre 200 000 et 5 000 000 Daltons, en particulier entre 250 000 et 800 000 Daltons, dans laquelle ladite masse moléculaire est la masse moléculaire moyenne de l'acide hyaluronique sans considération de la contribution de la masse de la riboflavine.

3. Composition pour une utilisation selon la revendication 1 ou 2, dans laquelle dans ledit conjugué d'acide hyaluronique, le degré de substitution de la riboflavine dans les esters est compris entre 2 et 7 %.

4. Composition pour une utilisation selon l'une quelconque des revendications précédentes, dans laquelle ledit acide hyaluronique utilisé pour la conjugaison avec la riboflavine est un acide hyaluronique obtenu par synthèse bactérienne, un acide hyaluronique d'origine animale, un acide hyaluronique réticulé, ou leurs combinaisons.

5. Composition pour une utilisation selon l'une quelconque des revendications précédentes, sous la forme d'un gel, d'une suspension, d'une solution, d'une capsule, d'un comprimé, d'une pilule, de granules, d'une poudre, de gouttes oculaires, d'un shampooing, d'une lotion, d'une charge, d'un spray, d'un bain de bouche.

6. Procédé pour la préparation du conjugué d'acide hyaluronique de l'une quelconque des revendications 1 à 4, comprenant une étape d'activation des groupes carboxy de l'acide hyaluronique avec un carbodiimide (EDC).

7. Trousse à usage intradermique comprenant la composition de la revendication 1 sous la forme d'un gel, d'une suspension ou d'un liquide, et une ou plusieurs seringues et/ou une ou plusieurs aiguilles.
